# EUROPÄISCHE PATENTSCHRIFT

(11) **EP 3 403 474 B1**
(45) Veröffentlichungstag und Bekanntmachung des Hinweises auf die Patenterteilung: **18.09.2019**
(21) Anmeldenummer: 16831612.3
(22) Anmeldetag: 16.12.2016
(51) Int. Cl.: H05H 1/24, A61L 2/14

(54) **GERÄT ZUR BEHANDLUNG EINER FLÄCHE MIT EINEM DIELEKTRISCH BEHINDERTEN PLASMA**
DEVICE FOR TREATING A SURFACE WITH A DIELECTRIC BARRIER PLASMA
DISPOSITIF DE TRAITEMENT D'UNE SURFACE AVEC UN PLASMA À BARRIÈRE DIÉLECTRIQUE

(30) Priorität: 13.01.2016 DE 102016100466
(43) Veröffentlichungstag der Anmeldung: 21.11.2018
(73) Patentinhaber: Cinogy GmbH, 37115 Duderstadt (DE)
(72) Erfinder: TRUTWIG, Leonhard, 37115 Duderstadt (DE); HAHNL, Mirko, 37339 Berlingerode (DE); STORCK, Karl-Otto, 37115 Duderstadt (DE); WANDKE, Dirk, 37308 Heilbad Heiligenstadt (DE); KOPP, Matthias, 37434 Gieboldehausen (DE)
(74) Vertreter: Gramm, Lins & Partner Patent- und Rechtsanwälte PartGmbB
(86) Internationale Anmeldenummer: PCT/DE2016/100591
(87) Internationale Veröffentlichungsnummer: WO 2017/121421

(56) Entgegenhaltungen:
- DE-A1-102012 103 470
- DE-A1-102013 019 058
- DE-B4-102013 000 440
- KR-A- 20110 002 210
- US-A1- 2008 183 167

## Beschreibung

Die Erfindung betrifft ein Gerät zur Behandlung einer Fläche mit einem dielektrisch behinderten Plasma, wobei die Fläche als Gegenelektrode fungiert, mit einem aus einem Gehäusekörper und einem Gehäusekopf gebildeten, elektrisch isolierenden Gehäuse, in dem sich eine Hochspannungszuleitung und eine Lagerung für eine mit der Hochspannungszuleitung verbundene Elektrode mit einem die Elektrode gegenüber der Fläche abschirmenden Dielektrikum befindet, wobei die Elektrode in dem Gehäusekopf drehbar gelagert ist und mit einem von dem Dielektrikum bedeckten Oberflächenabschnitt aus dem Gehäusekopf herausragt.

Es ist seit längerer Zeit bekannt, dass Oberflächen von Körpern durch eine Plasmabehandlung für bestimmte Anwendungszwecke vorteilhaft beeinflusst werden können. So ist es möglich, den Auftrag von Farben oder anderen chemischen Behandlungsmitteln auf einer Oberfläche zu erleichtern, die ohne eine entsprechende Vorbereitung in der gewünschten Form nicht bearbeitbar wäre. Die ursprünglich vorgesehene Ausbildung eines heißen Plasmas an derartigen Oberflächen setzt aufwändige Apparaturen und strenge einzuhaltende Sicherheitsvorkehrungen voraus, da Bedienpersonen sorgfältig von der das Plasma erzeugenden Hochspannung und von dem heißen Plasma selbst abgeschirmt werden müssen.

Es hat sich gezeigt, dass die Behandlung von Oberflächen mit einem dielektrisch behinderten Plasma möglich ist, dass zwar auch mit einer Hochspannung, insbesondere einer Wechsel-Hochspannung generiert wird, jedoch als kaltes Plasma nicht vor Bedienpersonen geschützt werden muss. Insbesondere hat sich herausgestellt, dass auch Oberflächen von lebenden Körpern, also Haut- und Wundoberflächen, mit einem dielektrisch behinderten Plasma in vorteilhafter Weise behandelt werden können, weil das Plasma beispielsweise eine zuverlässige Desinfektion auch in schwer zugänglichen Bereichen der Oberfläche ermöglicht und darüber hinaus die Hautoberfläche für die Aufnahme pflegender oder heilender Substanzen vorbereiten und die Wundheilung im Allgemeinen beispielsweise durch Erhöhung der Mikrozirkulation im Gewebe, positiv beeinflussen kann.

Es hat sich herausgestellt, dass eine sichere und effektive Ausbildung des Plasmas dadurch möglich ist, dass der zu der zu behandelnden Oberfläche gehörende Körper als Gegenelektrode (so genannte "floatende Elektrode") verwendet wird. Dies hat für das Behandlungsgerät zur Folge, dass es lediglich eine - die Hochspannung führende - Elektrode aufweist und keine eigene Gegenelektrode benötigt.

Ein Gerät zur Behandlung einer Fläche mit einem dielektrisch behinderten Plasma ist durch DE 10 2007 030 915 A1 bekannt. Eine längliche Elektrode mit einem zylindrischen Querschnitt und einer abgerundeten Stirnseite ist dabei von einem entsprechend ausgebildeten Dielektrikum umgeben. Mit der Mantelfläche des Dielektrikums kann eine Fläche, beispielsweise ein Hautareal, behandelt werden. Eine gleichmäßige Behandlung einer größeren Fläche ist mit einer derartigen Vorrichtung nicht vorgesehen.

Durch DE 10 2009 060 627 ist für die Behandlung größerer Hautareale eine flächige Elektrodenanordnung vorgesehen, bei der eine flächige Elektrode durch ein flächiges Dielektrikum gegenüber einer zu behandelnden Fläche, insbesondere Hautfläche, abgeschirmt wird. Zur Anpassung an unregelmäßige Oberflächen ist sowohl die Elektrode als auch das Dielektrikum flexibel ausgebildet. Eine derartige flächige Elektrode kann auf die zu behandelnde Fläche aufgelegt werden, wobei das Dielektrikum strukturiert ausgebildet ist um zwischen Haut und Dielektrikum einen Luftzwischenraum zu belassen, in dem die Plasmaentladung stattfinden kann, wenn die zu behandelnde Fläche als Gegenelektrode verwendet wird. Nachteilig an dieser Anordnung ist die Großflächigkeit der Elektrodenanordnung, die vorzugsweise an einer stationären Einrichtung verwendbar ist und darüber hinaus die Behandlung von stark gekrümmten Flächen nur bedingt ermöglicht.

Um ein Gerät zur Behandlung einer Fläche mit einem dielektrisch behinderten Plasma zu ermöglichen, mit dem in einfacher Handhabung die Plasmabehandlung sowohl stark gekrümmter Flächen als auch größerer Flächenareale möglich ist, sind Geräte vorgeschlagen worden, die über die Oberfläche leicht führbar sind. Gemäß DE 10 2012 103 470 A1 soll über eine kugelförmige Elektrode, die sich in einem Gehäuse frei drehen kann, eine Behandlung einer Oberfläche mit einem dielektrisch behinderten Plasma erfolgen. Die Einkopplung einer Wechsel-Hochspannung soll über eine Koppelelektrode kapazitiv erfolgen. Dies hat zur Folge, dass der Stromfluss zur kugelförmigen Elektrode bereits dielektrisch behindert ist. Wenn die Elektrode ohne eine dielektrische Abschirmung auf der zu behandelnden Oberfläche anliegt, entsteht somit um den punktförmigen Kontakt zwischen der Kugel und der zu behandelnden Oberfläche ein ringförmiger Bereich, in dem sich das dielektrisch behinderte Plasma ausbilden kann. Wenn allerdings gemäß einem anderen Ausführungsbeispiel die kugelförmige Elektrode mit einer dielektrischen Schicht umhüllt ist, kann sich mit einem vernünftigen Aufwand kein Plasma mehr an der Oberfläche ausbilden. Die Behandlung von leitfähigen Oberflächen, bei der die Abschirmung der kugelförmigen Elektrode zur Vermeidung von Bogenentladungen notwendig ist, lässt sich mit der bekannten Vorrichtung somit nicht sinnvoll durchführen.

Ein Gerät der eingangs erwähnten Art ist durch DE 10 2013 019 058 A1 bekannt. In vorteilhafter Weise befindet sich die Elektrode in Form einer drehbare Kugel in einem Gehäusekopf, der rastend mit einem Gehäusekörper, der als Griff ausgebildet ist, verbunden wird. Die Hochspannungszuführung befindet sich in dem Gehäusekörper. Die Kontaktierung der Elektrode mit der Hochspannungszuführung erfolgt dadurch, dass der Gehäusekopf einen mit der Elektrode verbundenen Anschlussstift aufweist, der im montierten Zustand des Gehäuses in einen passenden Führungskanal im Griffteil hineinragt und dort mit der Hochspannungszuführung verbindbar ist. Die kugelförmige Elektrode ist ihrerseits mit dem Anschlussstift über eine Leiterschlaufe verbunden, sodass die Kugel, die mit einer Kugelabschnittsfläche aus einer stirnseitigen Öffnung des Gehäusekopfes herausragt, jeweils nur begrenzt drehbar ist. Zum Schutze der Verbindung der Leiterschlaufe mit der Kugel wird die Drehbewegung der Elektrode mechanisch durch Anschläge begrenzt. Die Oberfläche der Kugel ist nahezu vollständig mit einem Dielektrikum versehen, sodass die kugelförmige Elektrode in dem Bereich, in dem sie aus dem Gehäusekopf herausragen kann, in jeder Drehstellung durch das Dielektrikum abgedeckt ist. Das Dielektrikum weist eine strukturierte Oberfläche in Form zahlreicher Noppen auf, die zwischen sich Lufträume ausbilden, in denen das Plasma durch die Hochspannung der Elektrode auch dann entstehen kann, wenn das Dielektrikum der Elektrode auf der zu behandelnden Oberfläche - im Wesentlichen punktförmig - aufliegt. Dieses Gerät erfüllt in der Praxis vollständig die Behandlungsfunktion durch das Plasma. Als nachteilig wird gelegentlich jedoch angesehen, dass die Elektrode nur begrenzt drehbar ist, sodass die Führung der Elektrode über die zu behandelnde Oberfläche auch dadurch bestimmt wird, dass sich die kugelförmige Elektrode auf einem Behandlungsweg nicht mehr weiterdrehen lässt, sodass der Behandlungsweg zumindest um 90° abgewinkelt werden muss. Häufig wird in diesem Fall auch eine Hin- und Herbewegung ausgeführt.

Der Erfindung liegt demgegenüber die Aufgabe zugrunde, ein Gerät der eingangs erwähnten Art so auszubilden, dass die Führung der Elektrode über die zu behandelnde Oberfläche nicht durch einen begrenzten Drehwinkel der Elektrode beeinträchtigt wird, wobei eine einfache galvanische Kontaktierung der Elektrode mit der Hochspannung erhalten bleiben soll.

Zur Lösung dieser Aufgabe ist ein Gerät der eingangs erwähnten Art erfindungsgemäß dadurch gekennzeichnet, dass die Elektrode in einer ersten Drehachse angeordnete Drehlagerelemente aufweist, mit denen sie im Zusammenwirken mit Drehlagerelementen im Gehäusekopf um die erste Drehachse drehbar gelagert und mit der Hochspannungszuleitung verbunden ist, wobei die erste Drehachse eine Mittenachse der Elektrode ist, und dass der Gehäusekopf drehbar um eine winkelig zur ersten Drehachse stehende zweite Drehachse mit dem Gehäusekörper verbunden und mit einer Kontaktanordnung zur Aufrechterhaltung einer elektrischen Verbindung zwischen der Elektrode und der Hochspannungszuleitung während der Drehbewegung um die jeweilige Drehachse versehen ist.

Das erfindungsgemäße Gerät ermöglicht eine freie Bewegung über die zu behandelnde Fläche dadurch, dass die Elektrode selbst zwar nur um eine einzige Drehachse drehbar ist, der die Elektrode lagernde Gehäusekopf jedoch selbst um eine winkelig, vorzugsweise senkrecht, dazu stehende Drehachse gedreht werden kann. Wenn beide Drehbewegungen unbegrenzt eingerichtet sind, lässt sich jeder Behandlungsweg auf der zu behandelnden Fläche realisieren. Im Rahmen der Erfindung ist es allerdings auch möglich, aus konstruktiven Gründen zumindest eine der Drehbewegungen zu begrenzen, wodurch eine geringe Einschränkung von Behandlungswegen eintreten kann, jedoch weiterhin eine große Beweglichkeit der Elektrode über die zu behandelnde Fläche bei einer einwandfreien Kontaktierung der Elektrode gewährleistet werden kann. Bei dem erfindungsgemäßen Gerät wird die Kontaktierung mit der Hochspannung während der Drehbewegung um die jeweilige Drehachse aufrechterhalten, wobei vorzugsweise wenigstens ein Drehlager eine unbegrenzte Drehung ermöglicht. In einer Ausführungsform der Erfindung ist jedenfalls die Lagerung der Elektrode in dem Gehäusekopf so ausgebildet, dass die Elektrode mit dem Dielektrikum unbegrenzt um die Drehachse drehbar ist. Zu diesem Zweck bestehen die Drehlagerelemente der Elektrode und des Gehäusekopfes vorzugsweise aus Achsansätzen und entsprechende Aufnahmen, die aus elektrisch leitendem Material gebildet sind. Die Achsansätze können sich dabei an der Elektrode oder an dem Gehäusekopf befinden, wobei die zugehörigen Aufnahmen an dem entsprechenden anderen Teil angeordnet sind. In einer konstruktiv einfachen Ausführungsform befinden sich die Achsansätze an der elektrisch leitenden Elektrode. Vorzugsweise befindet sich auch die Elektrode in dem Gehäusekopf und ragt lediglich mit einem Oberflächenabschnitt der vollständig von dem Dielektrikum bedeckt ist, aus einer Öffnung des Gehäusekopfs heraus.

Da sich die Elektrode um eine feste Achse dreht, kann sie die Form einer Walze aufweisen, wie sie im grundsätzlichen Aufbau aus der DE 10 2013 000 440 B4 bekannt ist. Der vorzugsweise aus der Öffnung des Gehäusekopfes hervorragende Oberflächenabschnitt kann als gerader Zylinderabschnitt oder als zweidimensionale Wölbung ausgebildet sein. Der gerade Zylinderabschnitt führt zu einer linienförmigen Anlage des Oberflächenabschnitts an der zu behandelnden Fläche, während die zweidimensionale Wölbung zu einer punktförmigen Anlage führt, wenn die zu behandelnde Fläche als Ebene aufgefasst werden kann.

Die Elektrode kann unabhängig von ihrer Form flexibel ausgebildet sein, indem sie beispielsweise aus einem federelastischen Material besteht, das aus einem gewickelten Draht oder Drahtgeflecht bzw. Streckmetallgitter gebildet sein kann und von einem flexiblen Dielektrikum abgedeckt ist wie dies ebenfalls in DE 10 2013 000 440 B4 grundsätzlich beschrieben ist. Die Elektrode kann sich dadurch etwaigen Unebenheiten der zu behandelnden Fläche anpassen.

Die drehbare Lagerung des Gehäusekopfes in dem Gehäusekörper erfolgt zweckmäßigerweise über isolierende Kunststoffteile des Gehäusekörpers einerseits und des Gehäusekopfes andererseits. Diese Ausbildung hat den Vorteil, dass der Gehäusekopf an dem Gehäusekörper mittels einer Rastverbindung befestigbar ist, die durch entsprechend geformte Kunststoffteile leicht rotationssymmetrisch so ausführbar ist, dass der Gehäusekopf um eine feste Drehachse relativ zum Gehäusekörper drehbar gelagert ist.

Für die Herstellung der elektrischen Verbindung zwischen der Hochspannungszuleitung des Gehäusekörpers mit der Elektrode des Gehäusekopfes ist es in einer konstruktiv einfachen Ausführung zweckmäßig, wenn die Drehlagerelemente des Gehäusekopfes mit einem elektrisch leitenden zylindrischen Stift verbunden sind, der im montierten Zustand des Gehäuses in den Gehäusekörper mit einem freien Ende in die Hochspannung führende Kontaktanordnung drehbar hineinragt. Dabei ist es zweckmäßig, wenn der elektrisch leitende zylindrische Stift vor dem freien Ende mit einer Kunststoffhülse des Gehäusekopfes umgeben ist, die in eine Aufnahme des Gehäusekörpers drehbar einsteckbar ausgebildet ist, wobei der Eintauchweg der Kunststoffhülse in die Aufnahme länger ist als die Kontaktstrecke zwischen dem freien Ende des elektrisch leitenden Stifts und der Kontaktanordnung. Die Kontaktanordnung kann dabei zweckmäßigerweise durch eine elektrisch leitende Hülse gebildet sein, die zumindest über eine definierte axiale Länge mit einer radialen Vorspannung federnd an dem freien Ende des elektrisch leitenden Stifts anliegt.

Auch bei dem erfindungsgemäßen Gerät ist es zweckmäßig, wenn das die Elektrode abdeckende Dielektrikum eine strukturierte Oberfläche aufweist, die die Ausbildung von Lufträumen beim Anliegen an der zu behandelnden Oberfläche gewährleistet, in denen sich das Plasma ausbilden kann.

Eine steckbare Ausbildung des Gehäusekopfes in dem Gehäusekörper, bei der eine Rastverbindung hergestellt wird, ermöglicht die Ausbildung eines Kopfteils des Geräts, das nach der Behandlung leicht ausgewechselt werden kann, um so als Wegwerfteil oder als in einem Gerät leicht zu desinfizierendes Bauelement zu dienen. Insbesondere als einmal zu verwendendes Bauteil ist es wichtig, dass das Kopfteil einfach und preisgünstig aufgebaut ist. Ein derartiger Aufbau ist der nachfolgenden Beschreibung eines Ausführungsbeispiels der Erfindung zu entnehmen, das in der Zeichnung dargestellt ist. Es zeigen:
- Figur 1: einen Vertikalschnitt durch ein Ausführungsbeispiel eines erfindungsgemäßen Geräts mit einem in den Gehäusekörper eingesetzten Gehäusekopf;
- Figur 2: eine Ansicht auf eine Stirnseite des Geräts gemäß Figur 1;
- Figur 3: einen Horizontalschnitt entlang der Linie C-C in Figur 2;
- Figur 4: eine Explosionsdarstellung des Gehäusekopfs;
- Figur 5: einen Vertikalschnitt durch den Gehäusekopf gemäß Figur 4;
- Figur 6: einen um 90° gedrehten Vertikalschnitt durch den Gehäusekopf;
- Figur 7: einen Horizontalschnitt durch den Gehäusekopf;
- Figur 8: eine Darstellung der Anordnung zur Kontaktierung der Elektrode innerhalb des Kopfgehäuses;
- Figur 9: eine Ansicht des Kopfteils des Geräts mit dem aus dem Kopfgehäuse hervorstehenden Oberflächenabschnitt der Elektrode.

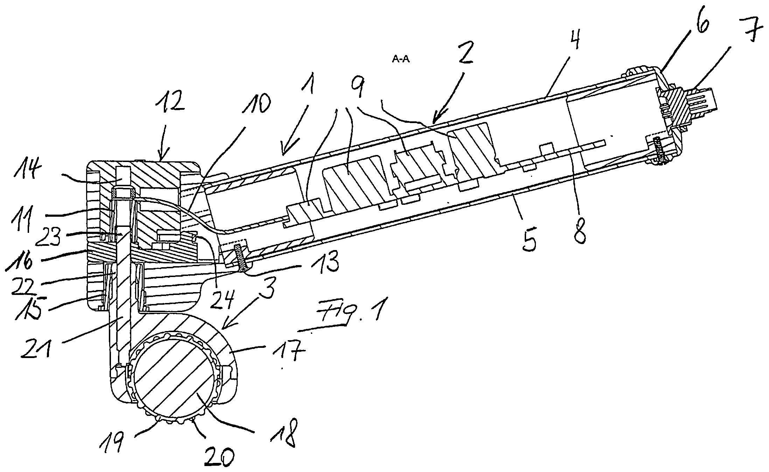

Das in Figur 1 dargestellte Gerät zur Behandlung einer Fläche mit einem dielektrisch behinderten Plasma weist ein Gehäuse 1 auf, das aus einem Griffteil 2 und einem Kopfteil 3 besteht, die leicht voneinander lösbar ausgebildet sind, sodass das Kopfteil 3 auswechselbar am Griffteil 2 anbringbar ist, um nach einer Behandlung durch ein neues Kopfteil 3 ersetzt zu werden. Dies ermöglicht insbesondere auch die Ausbildung eines Kopfteils, das nach der Behandlung desinfiziert oder entsorgt wird. Somit eignet sich das erfindungsgemäße Gerät insbesondere zur Behandlung der Haut oder einer Wundfläche eines lebenden Körpers. Die dabei durchgeführte Behandlung kann medizinischer oder kosmetischer Art sein.

Das Griffteil weist einen Gehäusekörper 4 auf, der einen langgestreckten zylindrischen Wandabschnitt 5 beinhaltet, durch den das Griffteil 2 mit einer Hand ergriffen werden kann. Der zylindrische Wandabschnitt ist an einem Stirnende, das vom Kopfteil 3 weg weist, durch eine Kappe 6 abgeschlossen, in die ein Stecker 7 eingesetzt ist, der zum Anschluss eines Stromversorgungskabels ausgebildet ist. Anstelle des Steckers 7 kann die Kappe 6 auch eine Kabeldurchführung für ein Stromversorgungskabel aufweisen. Innerhalb des Gehäusekörpers 4 befindet sich im Bereich des zylindrischen Wandabschnitts 5 eine Schaltungsplatine 8 mit darauf angeordneten Baugruppen 9, mit denen aus der zugeführten Versorgungsspannung, die eine Netzspannung sein kann, in bekannter Technik eine Hochspannung generiert wird. Hierzu kann beispielsweise die übliche Netzwechselspannung gleichgerichtet (vorzugsweise hochfrequent) zerhackt und auf die Hochspannung hochtransformiert werden. Am Ausgang der Baugruppen 9 steht dann eine Wechsel-Hochspannung, die über einen internen Leiter 10 auf ein Kontaktierungselement 11 geführt wird. In ähnlicher Technik kann die Versorgungsspannung auch einer Batterie oder einem Akkumulator entnommen und dem Zerhacker zugeführt werden. Batterie oder Akkumulator können im Griffteil 2 untergebracht sein, sodass eine Kabeldurchführung oder ein Stecker 7 nicht benötigt werden.

In einer alternativen Ausführungsform kann vorzugsweise über eine Kabeldurchführung durch die Kappe 6 bereits eine Hochspannung zugeführt werden, wodurch die Erzeugung der Hochspannung in dem Griffteil 2 entfallen kann. Aus Sicherheitsgründen ist jedoch die Zuführung einer üblichen Netzspannung und die Erzeugung der für die Plasmabehandlung benötigten Hochspannung in dem Gehäuse 1 bevorzugt.

Das Kontaktierungselement 11 befindet sich in einem Stirngehäuseteil 12 des Gehäusekörpers 4, das an das der Kappe 6 gegenüberliegende Ende des zylindrischen Wandabschnitts 5 fest angesetzt ist, beispielsweise mit wenigstens einer Schraube 13. Das Stirngehäuseteil 12 weist eine zum Kopfteil 3 hin offene Sackbohrung 14 auf, deren Achse mit einer gedachten Längsachse des zylindrischen Wandabschnitts 5 einen Winkel bildet, der etwas größer als 90° ist und vorzugsweise zwischen 100 und 120° liegt, sodass das Griffteil 3 zum freien Ende hin etwas nach oben verläuft, wenn die Sackbohrung 14 für die Behandlung der Fläche senkrecht zu der Fläche steht.

In die Sackbohrung 14 ist das Kontaktierungselement 11 in Form einer zylindrischen Hülse eingesetzt. Am unteren Ende der Sackbohrung 14 befindet sich ferner ein eingesetztes Kunststoffhülsenteil 15, das fest, beispielsweise durch Klebung, mit dem Stirngehäuseteil 12 verbunden ist. Zwischen dem Kontaktierungselement 11 und dem Kunststoffhülsenteil 15 weist das Stirngehäuseteil 12 eine Ausnehmung auf, in die ein Querschieber 16 eingesetzt ist, der eine Durchgangsöffnung aufweist, die in dem in Figur 1 dargestellten Zustand mit der Sackbohrung 14 fluchtet.

Figur 1 lässt ferner erkennen, dass in dem dargestellten montierten Zustand das Kopfteil 3 ein Kopfgehäuse 17 aufweist, in dem eine in dem dargestellten Ausführungsbeispiel kugelförmige Elektrode 18 drehbar gelagert ist, wie unten noch näher erläutert wird. Die Oberfläche der Elektrode 18 ist durch ein Dielektrikum 19 abgedeckt, dessen äußere Oberfläche eine Strukturierung in Form von zahlreichen Noppen 20 aufweist. In dem Kopfgehäuse 17 ist ein elektrisch leitender zylindrischer Stift 21 gelagert, der durch einen eine Kunststoffhülse 22 bildenden Ansatz des Kopfgehäuses 17 geführt ist und aus der Kunststoffhülse mit einem freien Ende 23 herausragt. Das nicht durch die Kunststoffhülse 22 abgedeckte freie Ende 23 des elektrisch leitenden zylindrischen Stifts 21 ragt in die das Kontaktierungselement 11 bildende Hülse im Stirngehäuseteil 12 hinein, wodurch die auf das Kontaktierungselement 11 übertragene Hochspannung auf den elektrisch leitenden zylindrischen Stift 21 übertragen wird. Dieser steht in unten noch näher erläuterter Weise in elektrischer Verbindung mit der Elektrode 18, wodurch die zur Erzeugung des Plasmas benötigte Hochspannung auf die Elektrode 18 gelangt.

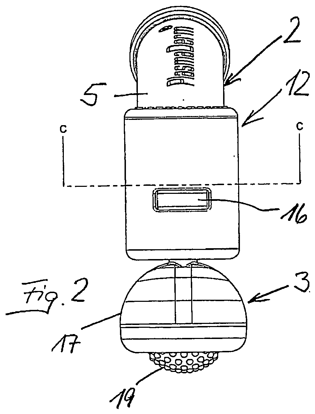

Der zylindrische Stift 21 durchläuft dabei eine Öffnung des Querschiebers 16, der mit Hilfe einer Feder 24 nach radial außen, also vom zylindrischen Wandabschnitt 5 weg, vorgespannt ist. Die Einführung des zylindrischen Stifts 21 in die Sackbohrung 14 bis zum Kontaktierungselement 11 gelingt somit nur dann, wenn der Querschieber 16 gegen die Kraft der Feder 24 in die in Figur 1 dargestellte Position geschoben wird. Wird das Kopfteil 3 aus dem Griffteil 2 entnommen, zieht die Feder 24 den Querschieber 16 nach außen, wodurch die Sackbohrung 14 durch einen massiven Abschnitt des Querschiebers 16 versperrt wird, sodass durch die Sackbohrung 14 vom offenen Ende her kein Zugang zu dem die Hochspannung führenden Kontaktierungselement 11 möglich ist. Der Querschieber 16 ragt in dieser Position nach Art einer Taste aus dem Stirngehäuseteil 12 heraus. Die Ansicht der Figur 2 verdeutlicht die Position des Querschiebers 16 in dem Stirngehäuseteil und die Lage des in das Griffteil 2 eingesetzten Kopfteils 3, bei dem ein Ober-flächenabschnitt der Elektrode 18 mit dem sie umgebenden Dielektrikum 19 aus einer Öffnung des Kopfgehäuses 17 nach unten herausragt. Der aus der Öffnung herausragende Oberflächenabschnitt liegt dabei deutlich unterhalb des größten Durchmessers der Elektrode 18, also deutlich unterhalb der Drehachse der Elektrode 18.

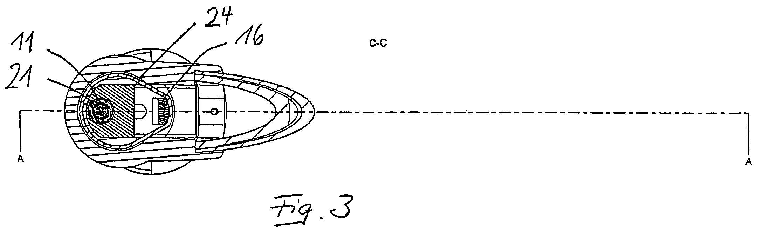

Figur 3 zeigt die Form der hier verwendeten Feder 24, die als geschlossene Bandfeder in dem Stirngehäuseteil 12 geführt ist und zum zylindrischen Wandabschnitt 5 des Gehäusekörpers 4 hin einen nach oben ragenden Ansatz des Querschiebers 16 umschlingt. Die Bandfeder 24 ist dabei elastisch ausgebildet und in dem in Figur 1 dargestellten gespannten Zustand aus einer Ausgangs-Kreisringform elastisch verformt, wodurch die nach außen gerichtete Vorspannung entsteht, die den Querschieber unter der Einwirkung der Feder 24 in eine die Sackbohrung 14 schließende Stellung zieht, wenn der zylindrische Stift 23 aus der Sackbohrung 14 herausgezogen wird.

Figur 1 lässt erkennen, dass die Kunststoffhülse 22 des Kopfgehäuses 17 und das sie im eigesetzten Zustand umgebende Kunststoffhülsenteil 15 komplementär geformt sind, um eine Rastverbindung zwischen dem Gehäusekörper 4 und dem Gehäusekopf 17 herzustellen, wodurch das Kopfteil 3 vom Griffteil 2 leicht entnehmbar ausgebildet ist, sodass es leicht ausgewechselt werden kann.

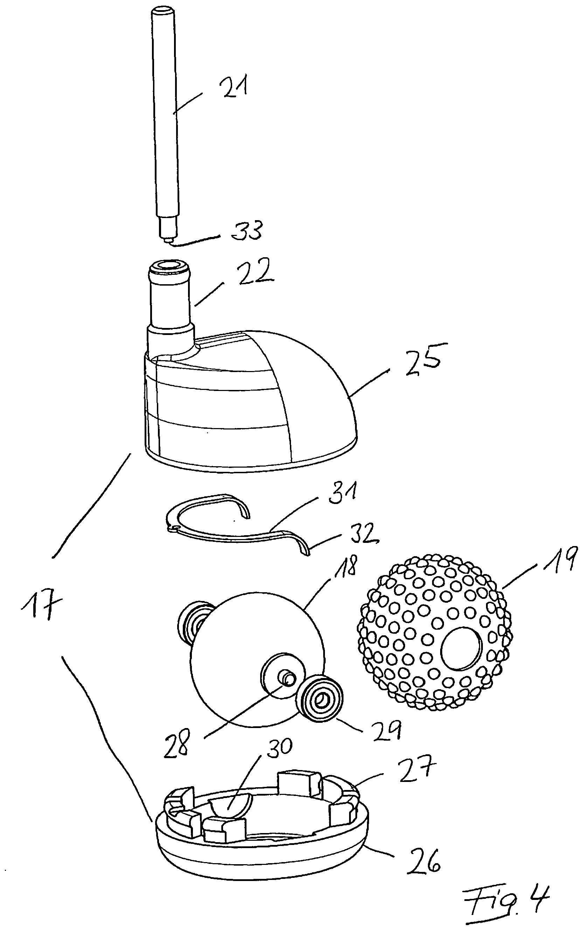

Figur 4 zeigt in einer explodierten Darstellung das Kopfteil 3 und lässt erkennen, dass der Gehäusekopf 17 zweiteilig ausgebildet ist und aus einem oberen Kalottenteil 25 und einem unteren Verriegelungsring 26 besteht, die durch Rastnasen 27 miteinander verbindbar sind. Die Stoßfuge zwischen dem Kalottenteil 25 und dem Verriegelungsring 26 befindet sich auf der Höhe des größten Durchmessers der in den Gehäusekopf 17 eingesetzten Elektrode 18 mit dem sie umgebenden Dielektrikum 19. Der Verriegelungsring 26 setzt die Kalottenform nach unten fort und bildet somit einen Kalottenabschnittsring, durch den die Elektrode in dem Gehäusekopf 17 gegen Herausfallen gesichert ist.

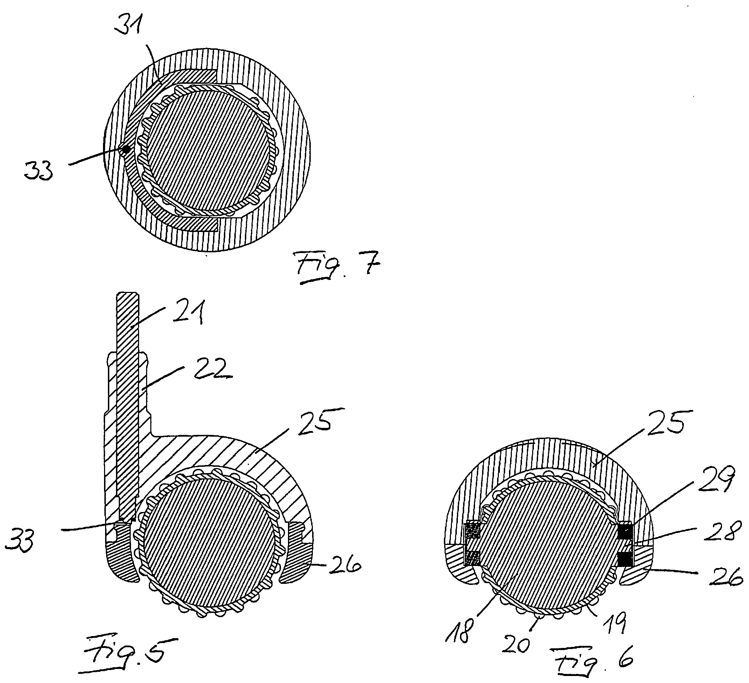

Für die drehbare Lagerung der Elektrode 18 ist diese mit diametral gegenüberliegenden Achsansätzen 28 versehen, die in einen Innenring eines Kugellagers 29 drehfest einsetzbar sind. Der Außenring des Kugellagers 29 wird drehfest in dem Gehäusekopf 17 gelagert, wofür an der Innenwandung entsprechende halbrunde Ausnehmungen 30 sowohl in dem Verriegelungsring 26 als auch im Kalottenteil 25 (nicht sichtbar) vorgesehen sind, die sich zu einer den Außenring des Kugellagers 29 aufnehmenden ringförmigen Ausnehmung zusammensetzen. Außen- und Innenring sowie die sich auf Außen- und Innenring abwälzenden Kugeln des Kugellagers 29 bestehen aus einem leitfähigen Werkstoff, insbesondere aus Stahl, sodass der Außenring des Kugellagers leitend mit den Achsansätzen 28 der Elektrode 18 - und damit mit der Elektrode selbst - verbunden ist. Mit den beiden Außenringen der Kugellager 29 kontaktiert ein halbringförmig ausgebildeter Federkontakt 31, in dem abgerundet abgewinkelte Enden 32 unter Vorspannung auf den Außenringen der Kugellager 29 aufliegen. Der Kontakt zwischen dem Federkontakt 31 und dem elektrisch leitenden zylindrischen Stift 21 wird dadurch hergestellt, dass der elektrisch leitende Stift 21 am unteren Ende als ein kleiner Bolzen 33 mit verringertem Durchmesser ausgebildet ist, der in eine entsprechende Öffnung 34 hineinragt und dort leitend mit dem Federkontakt 31 verbindbar ist. Die Schnittdarstellungen der Figuren 5, 6 und 7 verdeutlichen den Aufbau des montierten Gehäusekopfes 7 mit der darin gelagerten Elektrode.

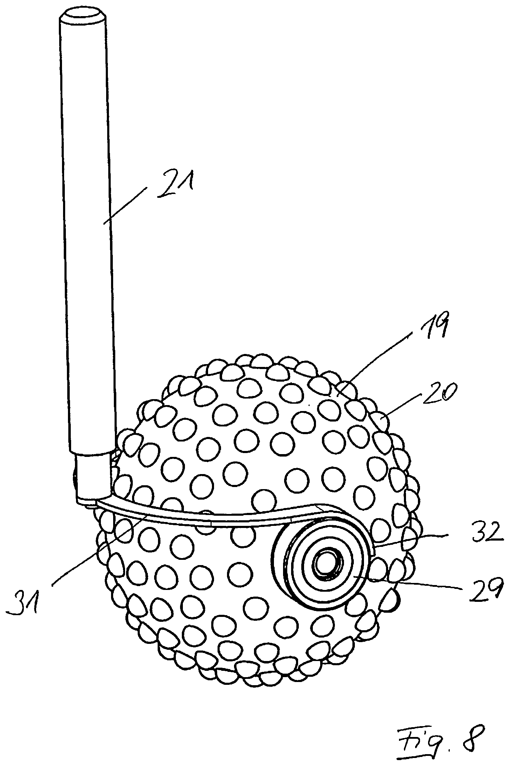

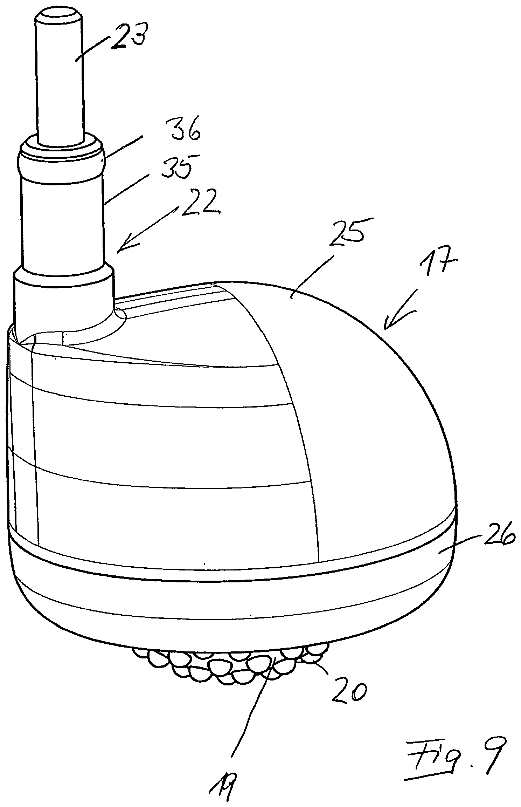

Figur 8 zeigt das Kontaktierungssystem für die Elektrode in einer gesonderten Darstellung ohne den tragenden Gehäusekopf 17. Figur 8 verdeutlicht dabei, dass die Achsansätze 28 durch das die Elektrode 18 umgebende Dielektrikum 19 in das Innere des Kugellagers 29 ragen. Die außerhalb des Dielektrikums 19 liegenden spannungsführenden Teile, nämlich Kugellager 29, Federkontakt 31 und zylindrischer Stift 21, werden durch den Gehäusekopf 17 isolierend abgedeckt. Daher ist es wichtig, dass nur ein deutlich unterhalb des Meridians der Kugel liegender Oberflächenabschnitt unten aus dem Gehäusekopf 17 herausragt, wie dies Figur 9 nochmals verdeutlicht. In Figur 9 ist ferner zu erkennen, dass die Kunststoffhülse 22, die den elektrisch leitenden zylindrischen Stift 21 unterhalb dessen freien Endes 23 umgibt, mit einer Rastnut 35 und einem Rastwulst 36 ausgebildet ist, die mit entsprechenden Gegenstücken in dem Kunststoffhülsenteil 15 des Stirngehäuseteils 12 die eine Drehung um die Mittenachse der Kunststoffhülse 22 bzw. des zylindrischen Stifts 21 ermöglichende Rastverbindung herstellen.

Figuren 8 und 9 verdeutlichen ferner die Ausführungsform der durch Noppen 20 strukturierten Oberfläche des Dielektrikums 19, durch die auch beim Anliegen der Noppenspitzen an der zu behandelnden Fläche Lufträume zwischen den Noppen 20 existieren, in denen sich aufgrund der der Elektrode 18 zugeführten Hochspannung relativ zu der zu behandelnden Fläche als Gegenelektrode das dielektrisch behinderte Plasma ausbilden kann. Allerdings ist die Ausführung der strukturierten Oberfläche mit Noppen nicht zwingend. Ebenso ist es möglich, die Oberfläche anders zu strukturieren, beispielsweise mit einer Gitterstruktur o. ä., da auch durch die Gitterstruktur abgeschlossene Lufträume eine Ausbildung des Plasmas ermöglichen.

Ferner verdeutlicht die Beschreibung des Ausführungsbeispiels für den Fachmann, dass die Elektrode 18 keineswegs in einer Kugelform vorliegen muss, da die Elektrode nur um die durch die Mitten der Achsansätze verlaufenden Drehachse drehbar ist, sodass für diese Drehbewegung auch eine walzenförmige Elektrode im Rahmen der Erfindung einsetzbar ist. Diese kann eine geradzylindrische Mantelfläche oder auch eine zweidimensional gewölbte Mantelfläche aufweisen. Sofern die Elektrode nur mit einem Oberflächenabschnitt aus einer (schlitzförmigen) Öffnung des Gehäusekopfs 17 herausragt, liegt dieser Oberflächenabschnitt vorzugsweise ebenfalls unterhalb der größten horizontalen Breite der walzenförmigen Elektrode, also deutlich unterhalb der beispielsweise durch Achsansätze 28 gebildeten Drehlagerelemente. Für die erfindungsgemäße freie Bewegung der Elektrode 18 über die zu behandelnde Fläche kann es zweckmäßig sein, die Länge der Walze im Verhältnis zum Durchmesser nicht zu groß zu wählen. Das Verhältnis Länge zu Durchmesser kann dafür ≤ 2:1, vorzugsweise ≤ 1,5:1 gewählt werden. Dies ist insbesondere für eine geradzylindrische und eine schwach gewölbte Mantelfläche vorteilhaft. Als schwach gewölbt wird eine Mantelfläche angesehen, deren Wölbungsradius in Längsrichtung der Walze mehr als doppelt so groß ist wie der größte radiale Durchmesser der Walze senkrecht zur Längsrichtung.

In dem dargestellten Ausführungsbeispiel stehen die Drehachsen für die Elektrode 18 einerseits und für den Gehäusekopf 17 andererseits senkrecht aufeinander, sodass bei winkelmäßig zur zu behandelnden Fläche unveränderter Position des Griffteils 2 eine freie Bewegung auf einer ebenen, zu behandelnden Fläche möglich ist. Bildet die Achse des Stifts 21 einen von 90° (geringfügig) abweichenden Winkel mit der Drehachse der Elektrode 18, wird eine Bewegung auf einer trichterförmigen Mantelfläche ausgeübt, was ebenfalls möglich ist, zumal durch die manuelle Führung des Geräts am Griffteil 2 jederzeit auch eine Veränderung der Position des Gehäuses 1 vorgenommen werden kann. In der Regel wird eine senkrechte Orientierung der beiden Drehachsen zueinander vorteilhaft sein.

## Patentansprüche

1. Gerät zur Behandlung einer Fläche mit einem dielektrisch behinderten Plasma, wobei die Fläche als Gegenelektrode fungiert, mit einem aus einem Gehäusekörper (4) und einem Gehäusekopf (17) gebildeten, elektrisch isolierenden Gehäuse (1), in dem sich eine Hochspannungszuleitung (10) und eine Lagerung für eine mit der Hochspannungszuleitung (10) verbundene Elektrode (18) mit einem die Elektrode (18) gegenüber der Fläche abschirmenden Dielektrikum (19) befindet, wobei die Elektrode (18) in dem Gehäusekopf (17) drehbar gelagert ist und mit einem von dem Dielektrikum (19) bedeckten Oberflächenabschnitt aus dem Gehäusekopf (17) herausragt,
**dadurch gekennzeichnet, dass** die Elektrode in einer ersten Drehachse angeordnete Drehlagerelemente (28) aufweist, mit denen sie im Zusammenwirken mit Drehlagerelementen (29) im Gehäusekopf (17) um die erste Drehachse drehbar gelagert und mit der Hochspannungszuleitung (10) verbunden ist, wobei die erste Drehachse eine Mittenachse der Elektrode (18) ist, und dass der Gehäusekopf (17) drehbar um eine winkelig zur ersten Drehachse stehende zweite Drehachse mit dem Gehäusekörper (4) verbunden und mit einer Kontaktanordnung (11, 21) zur Aufrechterhaltung einer elektrischen Verbindung zwischen der Elektrode (18) und der Hochspannungszuleitung (10) während der Drehbewegung um die jeweilige Drehachse versehen ist.

2. Gerät nach Anspruch 1, **dadurch gekennzeichnet, dass** die Elektrode (18) in dem Gehäusekopf (17) unbegrenzt drehbar gelagert ist.

3. Gerät nach Anspruch 1 oder 2, **dadurch gekennzeichnet, dass** die Drehlagerelemente (28, 29) der Elektrode (18) und des Gehäusekopfs (17) durch Achsansätze (28) und entsprechende Aufnahmen aus elektrisch leitendem Material gebildet sind.

4. Gerät nach einem der Ansprüche 1 bis 3, **dadurch gekennzeichnet, dass** der bei der Drehung der Elektrode (18) aus dem Gehäusekopf (17) herausragende Oberflächenabschnitt eine zweidimensionale Wölbung aufweist, sodass er zu einer punktförmigen Anlage an der zu behandelnden Fläche vorgesehen ist.

5. Gerät nach einem der Ansprüche 1 bis 3, **dadurch gekennzeichnet, dass** der bei der Drehung der Elektrode (18) aus dem Gehäusekopf (17) herausragende Oberflächenabschnitt als gerader Zylinderschnitt ausgebildet ist, der zu einer linienförmigen Anlage an der zu behandelnden Fläche vorgesehen ist.

6. Gerät nach einem die Ansprüche 1 bis 5, **dadurch gekennzeichnet, dass** die Elektrode (18) in dem Gehäusekopf (17) angeordnet ist und aus einer Öffnung des Gehäusekopfs (18) nur mit dem Oberflächenabschnitt herausragt.

7. Gerät nach einem der Ansprüche 1 bis 6, **dadurch gekennzeichnet, dass** der Gehäusekopf (17) in dem Gehäusekörper (4) unbegrenzt drehbar gelagert ist.

8. Gerät nach einem der Ansprüche 1 bis 7, **dadurch gekennzeichnet, dass** der Gehäusekopf (17) mit einem elektrisch leitenden zylindrischen Stift (21) versehen ist, der in dem Gehäusekörper (4) mit einem freien Ende (23) in eine die Hochspannung führende Kontaktanordnung (11) drehbar hineinragt.

9. Gerät nach Anspruch 8, **dadurch gekennzeichnet, dass** der elektrisch leitende zylindrische Stift (21) vor dem freien Ende (23) mit einer Kunststoffhülse (22) des Gehäusekopfs (17) umgeben ist, die in eine Aufnahme des Gehäusekörpers (4) drehbar einsteckbar ausgebildet ist, wobei der Eintauchweg der Kunststoffhülse (22) in die Aufnahme länger ist als die Kontaktstrecke zwischen dem elektrisch leitenden zylindrischen Stift (21) und der Kontaktanordnung (11).

10. Gerät nach Anspruch 8 oder 9, **dadurch gekennzeichnet, dass** die Kontaktanordnung (11) durch eine elektrisch leitende Hülse gebildet ist.

11. Gerät nach einem der Ansprüche 1 bis 10, **dadurch gekennzeichnet, dass** der Gehäusekopf (17) an dem Gehäusekörper (4) mittels einer Rastverbindung befestigt ist.

## Claims

1. A device for treating a surface with a dielectric barrier plasma, wherein the surface functions as a counter electrode, having an electrically insulating housing (1), which is formed from a housing body (4) and a housing head (17) and in which is situated a high voltage feed line (10) and a bearing arrangement for an electrode (18), which is connected to the high voltage feed line (10), with a dielectric (19) which shields the electrode (18) from the surface, wherein the electrode (18) is rotatably mounted in the housing head (17) and protrudes out of the housing head (17) by way of a surface portion which is covered by the dielectric (19), **characterized in that** the electrode comprises pivot bearing elements (28) which are arranged in a first pivot axis and by way of which, in conjunction with pivot bearing elements (29) in the housing head (17), it is mounted so as to rotate about the first pivot axis and is connected to the high voltage feed line (10), the first pivot axis being a center axis of the electrode (18), and **in that** the housing head (17) is connected to the housing body (4) so as to be rotatable about a second rotational axis, which is at an angle to the first rotational axis, and is provided with a contact arrangement (11, 21) for maintaining an electric connection between the electrode (18) and the high voltage feed line (10) during the rotation about the respective axis.

2. The device as claimed in claim 1, **characterized in that** the electrode (18) is rotatably mounted in a non-restricted manner in the housing head (17).

3. The device as claimed in claim 1 or 2, **characterized in that** the pivot bearing elements (28, 29) of the electrode (18) and of the housing head (17) are formed by axial projections (28), and corresponding receiving means produced from electrically conducting material.

4. The device as claimed in one of claims 1 to 3, **characterized in that** the surface portion which protrudes out of the housing head (17) when the electrode (18) is rotated comprises a two-dimensional arch such that it is provided for punctiform abutment against the surface to be treated.

5. The device as claimed in one of claims 1 to 3, **characterized in that** the surface portion which protrudes out of the housing head (17) when the electrode (18) is rotated is realized as a straight cylinder intersection which is provided for linear abutment against the surface to be treated.

6. The device as claimed in one of claims 1 to 5, **characterized in that** the electrode (18) is arranged in the housing head (17) and protrudes out of an opening of the housing head (18) only by way of the surface portion.

7. The device as claimed in one of claims 1 to 6, **characterized in that** the housing head (17) is rotatably mounted in a non-restricted manner in the housing body (4).

8. The device as claimed in one of claims 1 to 7, **characterized in that** the housing head (17) is provided with an electrically conducting cylindrical pin (21) which protrudes rotatably in the housing body (4) by way of a free end (23) into a contact arrangement (11) which carries the high voltage.

9. The device as claimed in claim 8, **characterized in that** the electrically conducting cylindrical pin (21) is surrounded in front of the free end (23) with a plastic sleeve (22) of the housing head (17) which is realized so as to be rotatably insertable into a receiving means of the housing body (4), wherein the push-in distance of the plastic sleeve (22) into the receiving means is longer than the contact section between the electrically conducting cylindrical pin (21) and the contact arrangement (11).

10. The device as claimed in claim 8 or 9, **characterized in that** the contact arrangement (11) is formed by an electrically conducting sleeve.

11. The device as claimed in one of claims 1 to 10, **characterized in that** the housing head (17) is fastened on the housing body (4) by means of a latching connection.

## Revendications

1. Appareil de traitement d'une surface avec un plasma empêché par voie diélectrique, la surface faisant office de contre-électrode, comportant un boîtier (1) d'isolation électrique formé par un corps de boîtier (4) et par une tête de boîtier (17), boîtier dans lequel se trouvent une ligne d'alimentation de haute tension (10) et un élément de montage pour une électrode (18) connectée à la ligne d'alimentation de haute tension (10) et pourvue d'un diélectrique (19) protégeant l'électrode (18) par rapport à la surface, l'électrode (18) étant montée mobile en rotation dans la tête de boîtier (17) et dépassant hors de la tête de boîtier (17) par une portion de surface recouverte par le diélectrique (19),
**caractérisé en ce que**
l'électrode comprend des éléments formant palier de rotation (28) agencés dans un premier axe de rotation, par lesquels elle est montée mobile en rotation autour du premier axe de rotation en coopération avec des éléments formant palier de rotation (29) dans la tête de boîtier (17) et est connectée à la ligne d'alimentation de haute tension (10), le premier axe de rotation étant un axe médian de l'électrode (18), et
**en ce que** la tête de boîtier (17) est reliée au corps de boîtier (4) en étant mobile en rotation autour d'un second axe de rotation disposé angulairement par rapport au premier axe de rotation, et est pourvue d'un ensemble de contact (11, 21) pour maintenir une connexion électrique entre l'électrode (18) et la ligne d'alimentation de haute tension (10), pendant le mouvement de rotation autour de l'axe de rotation respectif.

2. Appareil selon la revendication 1,
**caractérisé en ce que**
l'électrode (18) est montée mobile en rotation de façon illimitée dans la tête de boîtier (17).

3. Appareil selon la revendication 1 ou 2,
**caractérisé en ce que**
les éléments formant palier de rotation (28, 29) de l'électrode (18) et de la tête de boîtier (17) sont formés par des embouts d'axe (28) et par les logements correspondants en matériau électriquement conducteur.

4. Appareil selon l'une des revendications 1 à 3,
**caractérisé en ce que**
la portion de surface dépassant hors de la tête de boîtier (17) lors de la rotation de l'électrode (18) présente un bombement bidimensionnel, de manière à être prévue pour un appui ponctuel contre la surface à traiter.

5. Appareil selon l'une des revendications 1 à 3,
**caractérisé en ce que**
la portion de surface dépassant hors de la tête de boîtier (17) lors de la rotation de l'électrode (18) est réalisée sous forme de portion cylindrique prévue pour un appui linéaire contre la surface à traiter.

6. Appareil selon l'une des revendications 1 à 5,
**caractérisé en ce que**
l'électrode (18) est agencée dans la tête de boîtier (17) et ne dépasse que par la portion de surface hors d'une ouverture de la tête de boîtier (17).

7. Appareil selon l'une des revendications 1 à 6,
**caractérisé en ce que**
la tête de boîtier (17) est montée mobile en rotation de façon illimitée dans le corps de boîtier (4).

8. Appareil selon l'une des revendications 1 à 7,
**caractérisé en ce que**
la tête de boîtier (17) est pourvue d'une tige cylindrique (21) électriquement conductrice qui pénètre dans le corps de boîtier (4) par une extrémité libre (23) dans un ensemble de contact (11) menant la haute tension.

9. Appareil selon la revendication 8,
**caractérisé en ce que**
en avant de l'extrémité libre (23), la tige cylindrique (21) électriquement conductrice est entourée par une douille en matière plastique (22) de la tête de boîtier (17), qui est réalisée de manière à pouvoir être enfichée par rotation dans un logement du corps de boîtier (4), la distance de plongée de la douille en matière plastique (22) dans le logement étant plus longue que la longueur de contact entre la tige cylindrique (21) électriquement conductrice et l'ensemble de contact (11).

10. Appareil selon la revendication 8 ou 9,
**caractérisé en ce que**
l'ensemble de contact (11) est formé par une douille électriquement conductrice.

11. Appareil selon l'une des revendications 1 à 10,
**caractérisé en ce que**
la tête de boîtier (17) est fixée au corps de boîtier (4) au moyen d'une liaison par enclenchement.
